# EUROPEAN PATENT APPLICATION

(11) **EP 1 192 938 A2**
(43) Date of publication of application: **03.04.2002**
(21) Application number: 01308343.1
(22) Date of filing: 28.09.2001
(51) Int. Cl.: A61K 7/48

(54) **Composition to enhance permeation of topical skin agents**

(30) Priority: 29.09.2000 US 677511
(71) Applicant: JOHNSON & JOHNSON CONSUMER COMPANIES, INC., Skillman, NJ 08558 (US)
(72) Inventor: Liu, Jue-Chen, Belle Mead, NJ 08502 (US); Miller, Jonathan, Lawrenceville, NJ 08648 (US); Kung, John, Edison, NJ 08820 (US); Niemiec, Susan, Yardley, PA 19067 (US); Nystrand, Glenn, Lebanon, NJ 08833 (US)
(74) Representative: Fisher, Adrian John

(57) **Abstract**

This invention relates to compositions and methods for enhancing the penetration of topical skin agents into the skin wherein said compositions contain at least one active ingredient, a skin conditioner, a polymeric emulsifier and a botanical derivative.

## Description

This application is a continuation-in-part of patent application U.S. Serial No. 09/361,426 filed July 27, 1999, which is hereby incorporated herein by reference.

### 1. Field of the Invention

This invention relates to compositions and methods for enhancing the penetration of topical skin agents into the epidermal and dermal layers of the skin. More particularly, it relates to compositions containing at least one active ingredient, a skin conditioner or nutrient that can be enhanced and regulated in penetrating the skin with a polymeric emulsifier, and, alternatively, a sugar or a polyoxyethylene alcohol.

### 2. Background of the Invention

In the field of therapeutic skin care, topical agents are often applied to the skin. In order to ensure their therapeutic activity, these agents must be applied onto the skin and be allowed to penetrate the epidermis and dermis. Although conceptually simple, this has often proven to be a formidable task, because of the skin's intended function as a well-designed barrier to foreign matter from the ambient environment. The outermost layer of the skin is composed of the *stratum comeum*, or "horny layer", containing several layers of dead, keratinized and flattened skin cells. This layer is extremely difficult to penetrate. It contains approximately 15% water, 70% protein and 15% lipid. The predominant protein is keratin. In the stratum corneum, a cornified envelope forms around the keratin resulting in corneocytes. Between these corneocytes are the lipids that bind the corneocytes together. From this structure, two routes become available for active ingredients to enter the skin.

Hydrophobic active ingredient are generally expected to be more apt to penetrate the skin through the intercellular lipid spaces. Hydrophilic actives, however, are expected to penetrate the stratum corneum through a transcellular pathway, i.e., through the corneocyte. However, even though there are two routes of entry, most topical actives still have difficulty penetrating the stratum corneum. Furthermore, if a composition contains actives that are hydrophobic as well as hydrophilic, the known penetration enhancing agents for one type of active may not serve to assist the penetration of the other and, in fact, may be expected to inhibit such penetration.

Another problem arises when providing formulations that enhance the penetration of topical agents: increasing the amount of active agent in the skin often produces excessive skin irritation. This, of course, is extremely undesirable, particularly for patients who are suffering inflammatory skin diseases or conditions.

Therefore, an object of this invention is to provide a delivery system that enhances the skin penetration of topically active agents.

An additional object of this invention is to provide that such delivery system not only allows for enhancing the penetration of the active but regulating delivery of the topical active as well.

Yet another object of this invention is to provide a delivery system having a low irritation profile while enhancing the skin penetration of such active ingredients.

A novel composition that enhances the penetration of hydrophilic and/or hydrophobic topically active compounds through the outermost layer of the skin would be advantageous for delivering therapeutic agents to the skin. Surprisingly, we have found novel compositions that enhance and regulate the penetration of topical active ingredients. Moreover, the compositions of this invention are unexpectedly mild and non-irritating to the skin despite the increased penetration of topical active agents.

### Summary of the Invention

The novel compositions of this invention may enhance the penetration of either hydrophobic or hydrophilic topical active agents. The compositions of this invention further provide a method of enhancing the penetration of both hydrophobic and hydrophilic agents, as well as a method to regulate the penetration of such agents. The novel compositions of this invention that enhance the penetration of hydrophobic active agents contains at least one hydrophobic or hydrophilic active agent, and a polymeric emulsifier. This composition may more preferably contain a sugar.

The novel compositions of this invention that enhance the penetration of hydrophilic active agents may also contain at least one hydrophilic penetration-enhancing agent such as a polyoxyethylene alcohol. Additionally, other components that aid in enhancing and regulating the penetration of such topical active agent may be added to the compositions of this invention such as the following: a polymeric emulsifier, a sugar and a polyoxyethylene alcohol. Novel compositions of this invention that possess the property of enhanced penetration that contain a hydrophobic active agent may also contain at least one hydrophilic penetration-enhancing agent such as a sugar.

The novel compositions of this invention that provide the regulation of delivery of hydrophilic and hydrophobic active agents in the same composition contain at least one hydrophobic active agent; at least one hydrophilic active agent, such hydrophilic active agent optionally being a sugar, a sugar; and polyoxyethylene alcohol.

Polymeric emulsifiers, particularly those which have been hydrophobically-modified, are useful in the compositions of this invention. In both pharmaceutical and cosmetic compositions, lotions and creams have been used as popular delivery vehicles for applying topical actives. Emulsions are two-phase systems that contain two immiscible liquids, typically oil and water. In order to stabilize oil in water, ionic or non-ionic surfactants may be used to reduce interfacial surface tensions creating oil droplets dispersed in water. Unlike traditional emulsifiers, polymeric emulsifiers operate by creating gels around the oil droplets. When these droplets come near each other, they are repelled by the gel layers. Preferably, a nonionic polymeric emulsifier, more preferably a hydrophilic cross-polymer which has been hydrophobically modified and most preferably, a hydrophobically-modified polyacrylic acid emulsifier having from about 10 to about 30 carbon atoms is used in the products and compositions of this invention. Most preferably, the polymeric emulsifier should be Pemulen*, an acrylate/C10-30 alkyl acrylate crosspolymer commercially available from B.F. Goodrich Specialty Chemicals of Cleveland, Ohio. Surprisingly, delivery systems containing lipophilic topical active ingredients formulated in the compositions of this invention in conjunction with Pemulen* provided enhanced penetration of the lipophilic topical active ingredient. Preferably, the polymeric emulsifier should be present in the compositions of this invention an amount of from about 0.01 to about 20% by weight of the composition. More preferably, they should be present in an amount of from about 0.1 to about 5 weight percent of the composition. Most preferably, they should be present in an amount of from about 0.1 to about 1 weight percent of the composition.

Sugars have also been commonly used in pharmaceutical and cosmetic compositions as humectants. Surprisingly, in the compositions of this invention, sugars that were incorporated into such compositions for the purpose of improving the compositions' skin feel characteristics, served to enhance the penetration of hydrophobic topical active ingredients. We also found, surprisingly, that the combination of hydrophobically-modified polymeric emulsifiers and sugars enhanced the penetration of the hydrophobic active ingredients together to a greater degree than either would if used separately. Moreover, sugars that assist in enhancing penetration may be hydrophilic topically active agents themselves. Sugars that may be useful in the compositions of this invention include, for example, ascorbic acid-2-glucoside, oligosaccharides such as lactose and melibiose and the like. Preferably, the sugar should be present in the compositions of this invention an amount of from about 0.01 to about 20% by weight of the composition. More preferably, they should be present in an amount of from about 0.1 to about 10 weight percent of the composition. Most preferably, they should be present in an amount of from about 0.1 to about 7 weight percent of the composition.

In order to enhance the penetration of hydrophilic topical actives, a polyoxyalkylene alcohol may be incorporated into the compositions of this invention. More preferably, a polyoxyethylene alcohol may be incorporated into the compositions of this invention. More preferably, such alcohols as steareth-10-20 and the like may be incorporated into the compositions of this invention. Preferably, the polyoxyalkylene alcohol should be present in the compositions of this invention an amount of from about 0.01 to about 20% by weight of the composition. More preferably, they should be present in an amount of from about 0.01 to about 5 weight percent of the composition. Most preferably, they should be present in an amount of from about 0.01 to about 2 weight percent of the composition.

In a system that contains both the hydrophobically modified acrylic acid, sugar and polyoxyethylene alcohol, unexpectedly, the compositions not only increase permeation of the topical active ingredients, but can be used to regulate the penetration of the active ingredients as well. For example, by changing the ratios of the ingredients, either hydrophobic or hydrophilic active agent penetration may be up- or down-regulated in order to enhance the therapeutic benefits of the formulations of this invention. By balancing the proportions of the elements of the compositions of the invention, proper concentrations of topical actives could be delivered, depending upon the type of benefit desired. For example, a retinoid such as retinol may be utilized in a composition to combat wrinkles and prevent photodamage while ascorbic acid-2-glucoside may be utilized for the purpose of promoting even skin tone or preventing sun-induced erythema. Therefore, under some circumstances, the retinol benefit may be up-regulated in order to provide treatment of wrinkles while the penetration into the skin of another undesirable hydrophilic component that functions as a formulation excipient (e.g. disodium EDTA that causes irritation) may be down-regulated to achieve maximum benefit. Surprisingly, although increased penetration of actives occurred, irritation was found to be minimal.

Thus, for example, in a composition wherein a hydrophobic active ingredient is desired to be delivered to a great extent into the skin and the penetration of an irritating hydrophilic excipient is desired to be down-regulated, the ratio of the hydrophobically modified acrylic acid, sugar and polyoxyethylene alcohol present in such a composition should be from about 0.001 to about 1000. The ratio of the hydrophobically modified acrylic acid to the sugar should be from about 0.001 to about 1000. The ratio of the hydrophobically modified acrylic acid to the polyoxyalkylene alcohol should be from about 0.001 to about 1000. The ratio of the sugar to the polyoxyalkylene should be from about 0.001 to about 1000. More preferably, the ratios should be as follows: the ratio of the hydrophobically modified acrylic acid, sugar and polyoxyethylene alcohol present in such a composition should be from about 0.1 to about 10. The ratio of the hydrophobically modified acrylic acid to the sugar should be from about 0.1 to about 10. The ratio of the hydrophobically modified acrylic acid to the polyoxyalkylene alcohol should be from about 0.1 to about 10. The ratio of the sugar to the polyoxyalkylene should be from about 0.1 to about 10.

The compositions of this invention assist in enhancing skin penetration of hydrophobic, also known as lipophilic, compounds. More particularly, hydrophobic vitamins such as retinol and tocopherol and the like may be incorporated into the compositions of this invention as active agents. To maximize the delivery of a lipophilic agent, the composition contains at least one topical active agent and a hydrophilic polymer that has been hydrophobically modified. The use of a sugar in combination with the hydrophobically-modified hydrophilic polymer unexpectedly further increases the delivery of the active agent.

The addition of polyoxyalkylene alcohol should increase the penetration and regulation of any hydrophilic ingredients in the composition. Despite the enhanced penetration of the topical agents, the composition is surprisingly non-irritating to the skin.

Any topical dosage form known to those of ordinary skill in the art, including, but not limited to, lotions, gels, sprays, aerosols and mousses.

The compositions of this invention should preferably contain:
(a) a topically active amount of a pharmaceutical or cosmetic active ingredient;
(b) from about 0.01% to about 20% of a non-ionic polymeric emulsifier;
(c) optionally, from about 0.01% to about 20% of a sugar, and
(d) optionally, from about 0.01% to about 20% of a polyoxyethylene alcohol.

### Detailed Description of the Preferred Embodiments

This invention provides compositions and methods to enhance and regulate the delivery of topical agents. The compositions of this invention comprises a pharmaceutical agent or cosmetic active ingredient, hydrophilic polymer that has been hydrophobically modified, optionally a sugar, optionally, polyoxyalkylene alcohol or any combination thereof. The pharmaceutical active includes any drug, hydrophobic or hydrophilic in nature, that would be appropriate for topical use. The cosmetic active includes any ingredient appropriate for cosmetics, nutrients or skin conditioners. These compositions are also non-irritating to the skin.

The pharmaceutical actives that can be used in the compositions of this invention, but not limited to, are antimicrobials, allergy inhibitors, anti-acne, analgesics, antitussives, antipruritics, anesthetics, antihistamines, anti-infective agents, inflammation inhibitors, anti-emetics, anticholinergics, vasoconstrictors, vasodilators, and wound healing promoters and the like.

The cosmetic active ingredients that can be used in the compositions of this invention, but not limited to, are vitamins (e.g., vitamin B complex; including thiamine, nicotinic acid, biotin, pantothenic acid, choline, riboflavin, vitamin B6, vitamin B12, pyridoxine, inositol, carnitine; vitamins A, C, D, E, K and their derivatives, pro-vitamins), amino acids and their derivatives, herbal extracts, retinoids, flavonoids, antioxidants, anti-inflammatory, skin conditioners, skin lighteners, chelating agents, cell turnover enhancers, coloring agents, fragrances, pigments and sunscreens and the like. Preferably, the compositions of this invention also contain botanical derivatives which have effect upon the appearance or condition of the skin.

Botanical derivatives, where used in compositions of the present invention, may be derived from the following, non-limiting botanical families: leguminosae, solanaceae, gramineae, and cucurbitacea. Preferably, the compositions of this invention contain botanical derivatives from the leguminosae family, with particularly useful species including soybean, limabean, and blackbean. Preferably, the botanical derivatives contain naturally-occurring proteins and may also include isoflavones and derivatives therefrom, for example, daidzin, daidzein, genistin, and genistein and the like. Such derivatives may be in the form of extracts but are not limited to those forms.

Novel compositions of this invention may preferably include soy products that may be in the form of a fluid (e.g., soy milk) or a solid (e.g., soybean powder or soy milk powder). Likewise, derivatives of other botanicals may be in fluid or solid form. What is meant by "soy product" is a substance derived from a soybean. In one preferred embodiment, the soy product is preferably a non-heat treated soy product. What is meant by "non-heat treated soy product" is a soy product in which the processing temperature is not greater than about 55 degrees Celsius (as soy products processed above this temperature have a tendency to lose their active potential). The soy products of this invention may contain soy proteins, isoflavones, and combinations of the two.

The soy products which may be included in the compositions of this invention may be of a variety of forms and made through a variety of methods. Soy milk, for example can be made from fresh soybeans that have been previously harvested. One method for making soy milk is to soak the soybeans in deionized or purified water for several hours, and grind them after they are hydrated, with the addition of small quantities of water. The grinding process allows the soybean milk to be extracted. After collection, the soybean milk may be filtered to remove any residual parts of the bean and/or bean husk. Soy milk used in compositions of this invention can be fresh soy milk as described above, or may be made from soybean powder and water. Soybean powder is milled from soybeans and may also be lyophilized, spray dried, or freeze-dried, and the resulting soy milk may or may not be filtered. Another example is the use of soy milk powder, made from lyophilized, spray dried or freeze-dried soy milk, with the addition of water and finished with or without filtration or homogenization. Other methods of soybean extraction could also be used to create active ingredients used in this invention, such as through an ethanol/water extraction.

Compositions of the present invention may contain from about 1 to about 90%, by weight, of soy products. The soy products useful in this invention may be produced from all soybean species, regardless of their geographic origin, environmental exposure, harvest time and the like. The soybean products useful in compositions of this invention may have a distinctive odor, which is considered tolerable in some cultures, but is undesirable in others. If necessary, the odor of compositions of this invention may be reduced by using soybean products derived from specific strains of soybeans known to produce reduced odor, including, but not limited to, lipoxygenase-2-deficient beans and those having a modified sugar profile, and the like. A process to reduce oxygen levels in the formulation may also reduce the odor. Various masking agents or fragrances may also be used to mitigate the odor.

Preservatives may be used to prevent any microbial attack on the soy products. Examples of preservatives include phenoxyethanol and parabens wuch as methylparaben, ethylparaben, and propylparaben. Antioxidants may also be used to increase shelf life and stability of compositions comprising soy products.

Useful sunscreens, or ultraviolet screening agents, in compositions of this invention include filters for ultraviolet light having wavelengths in the UV-A range (from about 320 to about 400 nanometers) and the UV-B range (from about 280 to about 320 nanometers). Examples of such sunscreen agents include otctyl methoxycinnamate, methyl benzylidene camphor, octyl salicylate, phenylbenzidazole sulfonic acid, oxybenzone, aminobenzoic acid, homosalate, octocrylene, titanium dioxide, zinc oxide, dibenzolymethane, padimate O, trolamine salicylate, dioxybenzone, methyl anthanilate, derivatives thereof, and combinations thereof as well as others known to those of skill in the art. A particularly useful sunscreening system of compositions of this invention includes a combination of octyl methoxycinnamate and a derivative of dibenzolymethane.

Preferably, the hydrophobically-modified hydrophilic polymeric emulsifiers used in the compositions of this invention are hydrophobically modified acrylic acids. Such as akylacrylates and the esters. The akyl chain lengths ranges from C2-C30.

Sugars that can be used in the compositions of this invention may include, but are not limited to, glucose, oligosaccharides, more particularly disaccharides such as fructose, melibiose, xylose, sucrose, arbutin, maltose, glucosides glycosides and derivatives thereof and the like. Sugars function in the compositions of this invention to enhance penetration of both hydrophobic and hydrophilic active ingredients.

Polyoxyethylene alcohols function in the compositions of this invention to enhance the penetration of hydrophilic active ingredients and can be used in the compositions of this invention. Such polyoxyethylene alcohols include, but are not limited to: ceteths, laureths, myreths, oleths, steareths and trideths. One particularly preferred example is steareth-10 or Brij 76 made by ICI Surfactants of Delaware, USA.

The delivery system and active ingredients are incorporated in a pharmaceutically or cosmetically acceptable vehicle. Preferably, the pH of the compositions of this invention should be from about 5 to about 9, more preferably from about 5 to about 7.

Of course, topical skin care agents known to those of ordinary skill in the art may be incorporated into the compositions of this invention, including mineral oils, animal oils, vegetable oils and silicones have all been used in cosmetic creams and lotions of the emulsion type. In addition to such oils, other emollients and surface active agents have been incorporated in the emulsions, including glyceryl trioleate, acetylated sucrose distearate, sorbitan trioleate, polyoxyethylene (1) monostearate, glycerol monooleate, sucrose distearate, polyethylene glycol (50) monostearate, octylphenoxypoly (ethyleneoxy) ethanol, deacylerin penta-isostearate, sorbitan sesquioleate, hydroxylated lanolin, lanolin, triglyceryl diisostearate, polyoxyethylene (2) oleyl ether, calcium stearoyl-2-lactylate, methyl glucoside sesquistearate, sorbitan monopalmitate, methoxy polyethylene glycol-22/dodecyl glycol copolymer (Elfacos E200), polyethylene glycol-45/dodecyl glycol copolymer (Elfacos ST99), polyethylene glycol 400 distearate and glyceryl stearate; alcohols, such as cetyl alcohol and lanolin alcohol; myristates, such as isopropyl myristate; cetyl palmitate; cholesterol; stearic acid; propylene glycol; glycerine, sorbitol and the like. Thickeners such as natural gums and synthetic polymers, as well as preservatives such as methylparaben, butyl paraben, propylparaben and phenyoxyethanol, coloring agents and fragrances also are commonly included in such compositions. Other active ingredients such as sunscreen materials and antimicrobial materials may be utilized in the compositions of the present invention provided that they are physically and chemically compatible with the other components of the compositions.

The following examples illustrate, but do not serve to limit the scope of the compositions and methods of this invention.

### Examples

### Example 1: Determination of Penetration and Regulation of Topical Agents

Five formulations were made containing the following ingredients:

### Formulation A (Comparison formulation):

| Ingredient | Weight Percent |
|---|---|
| Water | 73.86% |
| Thickeners | 1.35% |
| Chelating agent | 0.10% |
| Panthenol | 0.50% |
| Glycerine | 3.00% |
| Whitening agent | 3.00% |
| PH adjustor | 0.05% |
| C12-15 alkyl benzoate | 4.00% |
| Octyl hydroxy stearate | 1.00% |
| Dimethicone | 1.00% |
| Cetyl alcohol | 2.50% |
| Cetearyl glucoside | 1.40% |
| Tocopheryl acetate and Tocopherol | 0.55% |
| Sunscreen | 4.00% |
| Preservative | 1.25% |
| Stabilizers | 1.10% |
| Retinol | 0.04% |

The following formulations B, C, D and E were made as set forth below: Formulation B:

| Ingredient | Weight Percent |
|---|---|
| Water | 78.04% |
| Glycerin | 3.00% |
| D panthenol | 0.50% |
| Disodium EDTA | 0.10% |
| Preservative | 0.73% |
| Preservative | 0.35% |
| Acrylates/C10-30Alkyl | 0.25% |
| Acrylate Cross-Polymer Carbomer | 0.40% |
| Ascorbic Acid | 0.01% |
| Dibutylhydroxy-toluene | 0.10% |
| Cetyl Alcohol | 2.00% |
| C₁₂-₁₅ alkyl benzoate | 4.00% |
| Octyl hydroxy stearate | 1.00% |
| Dimethicone | 1.00% |
| Di-alpha tocopheryl acetate | 0.50% |
| Octyl methoxy-cinnamate | 4.00% |
| Propyl paraben | 0.17% |
| Na hydroxide (10%) | 2.60% |
| Retinol 50c | 0.20% |
| Tocopherol | 0.05% |
| Thea Sinesis Extract | 1.00% |

### Formulation C:

| Ingredeint | Weight Percent |
|---|---|
| Water | 73.39% |
| Glycerin | 3.00% |
| D panthenol | 0.50% |
| Disodium EDTA | 0.10% |
| Ascorbic Acid-2G | 2.00% |
| Phenoxyethanol | 0.73% |
| Methyl paraben | 0.35% |
| Xanthan gum | 0.20% |
| Hydroxyethylcellulose | 1.15% |
| Ascorbic Acid | 0.01% |
| Dibutylhydroxytoluene | 0.10% |
| Cetearyl glucoside | 1.40% |
| Cetyl Alcohol | 2.00% |
| C₁₂-₁₅ alkyl benzoate | 4.00% |
| Octyl hydroxy stearate | 1.00% |
| Dimethicone | 1.00% |
| Di-alphatocopheryl acetate | 0.50% |
| Octyl methoxycinnamate | 4.00% |
| Propyl paraben | 0.17% |
| Na hydroxide (10%) | 2.45% |
| Retinol 50c | 0.20% |
| Polyacrylamide & laureth 7 & C13-C14 isoparafin | 0.70% |
| Tocopherol | 0.05% |
| Thea Sinesis Extract | 1.00% |

### Formulation D:

| Ingredient | Weight Percent |
|---|---|
| Water | 72.82% |
| Glycerin | 3.00% |
| D panthenol | 0.50% |
| Disodium EDTA | 0.10% |
| Preservative | 0.73% |
| Preservative | 0.35% |
| Acrylates/C10-30 Alkyl Acrylate Cross-Polymer | 0.25% |
| Dimethicone | 1.00% |
| Cetyl Alcohol | 2.00% |
| Di-alpha tocopheryl acetate | 0.50% |
| Octyl methoxycinnamate | 4.00% |
| Propyl paraben | 0.17% |
| Na hydroxide (18%) | 1.50% |
| Retinol 50c | 0.18% |
| Ascorbic Acid-2G | 6.35% |
| Tocopherol | 0.05% |
| Thea Sinesis Extract | 1.00% |

### Formulation E:

| Ingredient | Weight Percent |
|---|---|
| Water | 71.59% |
| Glycerin | 3.00% |
| D panthenol | 0.50% |
| Disodium EDTA | 0.10% |
| Ascorbic Acid-2G | 2.00% |
| Preservative | 0.73% |
| Preservative | 0.35% |
| Acrylates/C₁₀₋₃₀ Alkyl Acrylate Cross-Polymer | 0.25% |
| Carbomer | 0.40% |
| Ascorbic Add | 0.01% |
| Dibutylhydroxy-toluene | 0.10% |
| Steareth-10 | 2.00% |
| Cetyl Alcohol | 2.00% |
| C₁₂-₁₅ alkyl benzoate | 4.00% |
| Octyl hydroxy stearate | 1.00% |
| Dimethicone | 1.00% |
| Di-alpha tocopheryl acetate | 0.50% |
| Octyl methoxycinnamate | 4.00% |
| Preservative | 0.17% |
| Na hydroxide (10%) | 5.05% |
| Retinol 50c | 0.20% |
| Tocopherol | 0.05% |
| Thea Sinesis Extract | 1.00% |

### Formulation F:

| Ingredient | Weight Percent |
|---|---|
| Water | 49.484 |
| Squalane | 15.000 |
| Glycerin | 10.000 |
| Macademia Nut Oil | 7.000 |
| Pentaerythritol Tetraoctanoate | 5.000 |
| Butylene Glycol | 4.000 |
| Petrolatum | 3.000 |
| Quaternium 18 Hectorite | 2.700 |
| Polyglyceryl-2-Diisostearate | 2.000 |
| PEG 150 | 1.000 |
| Retinol | 0.166 |
| Trisodium EDTA | 0.100 |
| Ascorbic Acid | 0.100 |
| Sodium Citrate | 0.100 |
| Tocopheryl Acetate | 0.100 |
| Preservative | 0.100 |
| Preservative | 0.100 |
| Butylated Hydroxytoluene (BHT) | 0.050 |

### Formulation G:

| Ingredient | Weight Percent |
|---|---|
| Purified Water | 71.294 |
| Gylcerin | 3.000 |
| Ascorbyl Gucoside | 2.000 |
| Phenoxyethanol | 0.800 |
| D Panthenol | 0.500 |
| Carbomer | 0.750 |
| Acrylates/C10-30 Alkyl Acrylate Crosspolymer | 0.250 |
| Preservative | 0.520 |
| Disodium EDTA | 0.200 |
| Ascorbic Acid | 0.010 |
| Sodium Hydroxide | 0.720 |
| C12-15 Alkyl Benzoate | 4.000 |
| Octyl Hydroxystearate | 1.000 |
| Octyl Methoxycinnamate | 7.500 |
| Avobenzone | 3.000 |
| Cetyl Alcohol | 1.000 |
| Dimethicone | 1.000 |
| Steareth-10 | 0.500 |
| BHT | 0.100 |
| Soybean (glycine soja) protein (and) Butylene Glycol (and) water | 0.500 |
| Tocopherol | 0.050 |
| Retinol (and) Polysorbate 20 | 0.206 |
| Iodopropynyl Butylcarbamate | 0.300 |
| Fragrance | 0.300 |

Formulation B was made by adding water to a beaker and overcharging the beaker with 20 grams of water. The water was then purged with argon or nitrogen gas. After 10-15 minutes, 20 grams of water was removed to check for oxygen content. If there was significant measurable oxygen in the sample, the purging was continued. Once oxygen was purged from the water, glycerin, panthenol, disodium EDTA, a first preservative and ascorbic acid were added to the beaker. The acrylates/C10-30 alkyl acrylate and carbomer were then added to the water phase. The beaker was then transferred to a vacuum close kettle homogenizer under yellow lights and any residual oxygen removed. The beaker was then heated to 70 - 75°C. A second preservative was added and mixing continued until it dissolved. The water phase was then neutralized with NaOH (10%) and the temperature held at 70 - 75°C for phasing. The remainder of the ingredients but for the Retinol, Tocopherol and Thea Sinesis Extract were combined in a separate beaker and heated to 70 - 75°C. When both phases were at the same temperature and homogenous, the oil phase was added to the water phase under vacuum and homogenized together. The beaked was then cooled slowly. Retinol was added when the temperature reached 55°C and Tocopherol and Thea Sinesis extract added at 45°C. Formulation C was made in a similar manner, except that AA-2G was added in addition to the ascorbic acid and, after the ascorbic acid was added, the xanthan gum, hydroxyethylene and glycerin were added to the water phase. Formulation D was made similarly to Formulation B. Formulation E was made similarly to Formulation C except that Steareth 10 was added to the oil phase.

Formulation F was prepared by combining water, glycerin, PEG150, and butylene glycol in a beaker, and heating it to 75°C. At 75°C, Trisodium EDTA, ascorbic acid and sodium citrate was added. Combining squalene, Mac. Nut oil, pentaerythritol tetraoctanoate, petrolatum, quaternium 18 hectoriate, polyglyceryl-2-diisostearate, and heating the mixture to 80°C while mixing. At 80°C, parabens and BHT were added to the mixture. The water phase was added to oil phase slowly and the heated was stopped. At 50°C, Vitamin E acetate and retinol were added. The whole process should be under argon and yellow light conditions.

Formulation G was prepared by separately preparing a water phase and an oil phase and then homogenizing the two phases. The water phase was prepared by first hydrating the carbomer with deionized water, followed by adding acrylate/C10-30 Alkyl Acrylate Copolymer. This mixture was moderately mixed and heated to a temperature of 75-80°C. Next the Glycerin, Ascorbyl Glucoside, Phenoxyethanol and D-Panthenol were added separately with mixing in between each ingredient addition. With the batch at a temperature of 75-80°C and in a mixing state, the following ingredients were added: Preservatives, Disodium EDTA and Ascorbic Acid. The temperature was brought back to 75-80°C and the Sodium Hydroxide was added. The composition was mixed under vacuum. The oil phase was prepared by adding the following ingredients: C12-15 Alkyl Benzoate, Octyl Hydroxystearate, Octyl Methoxycinnamte, Avobenzone, Cetyl Alcohol, Dimethicone and Steareth-10. This mixture was moderately mixed and heated to a temperature of 75-80°C. The oil phase was added to the water phase and mixed under a vacuum for 5-10 minutes at 75-80°C. Under yellow lights, the combined phases were cooled to 45°C, and the vessel containing the mixture was purged with Argon. The remaining ingredients (Soybean protein/Glycol/water mixture, Tocopherol, Retinol/Polysorbate 20, Iodopropynyl Butylcarbamate and Fragrance) were added separately with mixing in between each ingredient addition. Lastly, the batch was homogenized for 5 minutes, and mixed and cooled to 30°C.

Experiments were conducted to determine the enhanced penetration and regulation effect of the delivery system. To determine transdermal penetration, *in vitro* skin permeation studies were conducted using non-occluded Franz diffusion cells.

Human cadaver skin section were mounted in Franz diffusion cells containing a receptor medium composed of a phosphate buffer with 0.025% butylated hydroxytoluene and 1.5% oleth-20. The receptor capacity was 5 milliliters (ml) and the cell surface area was 0.636 cm². A 400 µm dose of one of the following formulations was applied to the diffusion cell. After 24 hours, the surface of the cells were cleansed with a solution of methanol and ethyl acetate. The epidermis and dermis were separated, chopped and placed into vials containing a solution methanol and ethyl acetate and subjected to sonication to fragment the skin. After sonication, the skin fragments were analyzed using HPLC. Samples were taken at zero and 24-hour time-points. Penetration of active ingredient was calculated based upon a percentage of applied dose. For these studies, the penetration of a lipophilic agent (retinol and a hydrophilic agent (ascorbic acid 2-glucoside, or "AA2G") were investigated.

The formulations investigated are set forth in Table 1 below:

**TABLE 1**

| | Composition | Ingredients | % of applied dose of retinol delivered into epidermis | Enhancement factor of retinol delivery | % of applied dose of AA-2G delivered into epidermis | Enhancement factor of AA-2G delivery |
|---|---|---|---|---|---|---|
| A | Conventional emulsifier (Control) | Cetearyl Glucoside | 0.175% | 1.00 | N/A | N/A |
| B | Hydrophobically modified acrylic acid emulsifier | Acrylates/C10-30 alkyl acrylate crosspolymer | 0.642% | 3.67 | N/A | N/! |
| C | Conventional emulsifier and sugar | Cetearyl glucoside and AA-2G | 0.241% | 1.38 | N/A | N/A |
| D | Hydrophobically modified acrylic acid and sugar | Acrylates/C10-30 alkyl acrylate crosspolymer and AA-2G | 1.25% | 7.20 | 0.18% | 1 |
| E | Hydrophobically modified acrylic acid, sugar and polyoxyethylene alcohol | Acrylates/C10-30 alkyl acrylate crosspolymer, AA-2G and steareth-10 | 0.464 | -2.70 | 1.016% | 5.65 |

From the above data, it can be seen that a control formulation (Formulation A) containing only cetearyl glucoside delivered only 0.175% of the applied dose of retinol into the epidermis. Surprisingly, however, when a formulation containing hydrophobically modified acrylic acid emulsifier was used (Formulation B), the percentage of retinol delivered increased to 0.642%, a 3.669 fold increase in delivery. When AA-2G and cetearyl glucoside were placed into formulation with retinol (Formulation C), the retinol permeation surprisingly increased to 0.241%, a 1.38-fold increase over the control formulation A. Even more surprisingly, a formulation containing both hydrophobically modified acrylic acid and AA-2G (Formulation D), although an additive effect was expected, a total delivery of retinol of 1.26% or a 7.2 fold increase in retinol delivery to the epidermis.

The activity of Formulation E demonstrates that the addition of a polyoxyethylene alcohol increased the penetration from 0.18% to 1.016%, or a 5.65-fold increase of delivery of AA-2G. Surprisingly, the retinol permeation decreased from 1.25% to 0.464%, a 0.36-fold decrease. Thus, the compositions of this invention afford a method of regulating the delivery of both hydrophilic and lipophilic ingredients.

### Example 2: Low Level of Irritation Demonstrated by Compositions of This Invention

Those of ordinary skill in the art of formulating topical skin care compositions would expect an increase in skin irritation to accompany an increase in penetration of active ingredients. The compositions of this invention, however, were surprisingly non-irritating despite the improved penetration of active ingredients.

A standard test for skin irritation, called the "Modified Irritation Study" (MIS) was used to evaluate the delivery system using retinol as the topical agent. This test measures the irritation potential of compositions in human volunteers. Test formulations of this invention were applied to fifty test subjects three times per week for three weeks for a total of nine applications. An occlusive patch with 0.2 to 0.3 gm of each test composition was applied to the upper back of the human subject. The patches remained in place for an initial 24 hours. After 24 hours, the subject would remove the patch from the back. A 24-hour rest period, during which no test material was applied, followed the removal of a Monday and Wednesday patch application. A 48-hour rest period followed a Saturday patch removal.

After each rest period, the test areas were observed by a study coordinator and graded according to a scale of 0 to 4.0. Fresh test material and patches were applied to the identical test sites until nine induction patches were completed.

The nine application scores for each test site for each subject were summed to yield a total score for 21 days. A grand total score for a test sample was obtained by adding the 21-day totals for all subjects. The grand total scores were normalized against the positive control (Formulation F below) , which received a normalized score of 100 to obtain a Normalized Irritation Score. The results of these tests are set forth in Table 2 below.

**TABLE 2**

| | Composition | Ingredients | Retinol Concentration | Total amount of retinol delivered (µg) | Normalized Irritation Score | Ratio of total retinol delivered:Norrna lized Irritation Score |
|---|---|---|---|---|---|---|
| A | Convention emulsifier (Control) | Cetearyl Glucoside | 0.04% | 0.21 | 9.88 | 2.1 |
| B | Hydrophobically modified acrylic acid emulsifier | Acrylates/C10-30 alkyl acrylate crosspolymer | 0.075% | 1.44 | 68.4 | 2.1 |
| C | Conventional emulsifier and sugar | Cetearyl glucoside and AA-2G | 0.075% | 0.54 | 46.5 | 1.2 |
| D | Hydrophobically modified acrylic acid and sugar | Acrylates/C10-30 alkyl acrylate crosspolymer and AA-2G | 0.075% | 2.84 | 21 | 13.5 |
| F | Water-in-oil emulsifier | Polyglyceryl-2-diisostearate and PEG 150 | 0.15% | N/A | 100 | N/A |

An increase in retinol penetration would generally be expected to result in higher skin irritancy, or a lower ratio of Total Retinol Delivered:Normalized Irritation Score. Ratios of the amount of retinol delivered to the irritation score were calculated to compare the formulations, i.e., they represent the amount of retinol delivered per each unit of irritation. As can be seen from the data set forth in Table 2, Formulations A, B and C are all comparable to each other. Formulation is a commercial product known to be mildly irritating. From these results with respect to Formulations A, B and C, it would appear that neither the hydrophobically modified acrylic acid emulsifier nor the sugar have an effect upon irritation mitigation.

However, surprisingly, Formulation D evidences a dramatic increase in retinol delivery per unit of irritation and, therefore, is considerably less irritating than Formulations A, B and C. We would also expect that the therapeutic index of Formulation D would be greater than that of Formulations A, B or C in light of the increased amount of retinol delivered at a lower extent of irritation. We conclude that the irritation mitigation effect is unexpectedly greater in compositions containing both hydrophobically modified acrylic acid and sugar.

Delivery of hydrophilic active ingredients using the compositions of this invention and the concomitant irritation mitigation effect may be seen exemplified below in Table 3.

**TABLE 3**

| | Composition | Ingredients | AA-2G Concentration | Total Amount AA-2G delivered (µg) | Normalized Irritation Score | Ratio of total AA-2G delivered: Normalized Irritation Score |
|---|---|---|---|---|---|---|
| D | Hydrophobically modified acrylic acid and sugar | Acrylates/C10-30 alkyl acrylate crosspolymer and AA-2G | 2% | 1.08 | 21 | 0.05 |
| E | Hydrophobically modified acrylic acid, sugar and polyoxyethylene alcohol | Acrylates/C10-30 alkyl acrylate crosspolymer, AA-2G and steareth-10 | 2% | 6.09 | 26 | 0.23 |
| F | Water-in-oil emulsifier | Polyclyceryl-2-diisostearate and PEG 150 | 0% | N/A | 100 | N/A |

As set forth above in Table 3, one unit of irritation results in the delivery of 0.05% AA-2G. With the addition of a polyoxyethylene alcohol, however, this number unexpectedly increases to 0.23%, meaning that more AA-2G is delivered to the skin with a lower irritation, generating a greater efficacy or therapeutic index.

### Example 3: Additional Formulations of the Invention

The compositions of this invention may be made by traditional preparation method. The following Table 4 illustrates compositions of this invention which we believe would serve to enhance the delivery of hydrophobic and hydrophilic active ingredients to the epidermis and dermis of the skin with relatively low levels of irritation.

**TABLE 4**

| Ingredient | Function | Formulation 1 | Formulation 2 | Formulation 3 |
|---|---|---|---|---|
| Water | Vehicle | q.s. 100% | q.s. 100% | q.s. 100% |
| Glycerin | Humectant | About 0 - about 10% | About 0 - about 10% | About 0 - about 10% |
| Disodium EDTA | Chelator | About 0- about 1% | About 0- about 1% | About 0- about 1% |
| Preservative | Preservative | About 0.1 to about 2 | About 0.1 to about 2 | About 0.1 to about 2 |
| Carbomer | Thickener | About 0.1 to about 1% | About 0.1 to about 1% | About 0.1 to about 1% |
| Pemulen | Hydrophobically modified polymer emulsifier | About 0.1 to about 1% | About 0.25% | About 0.25% |
| Ascorbic Acid 2-Glucoside | Sugar | 0% | About 0.1% to about 5% | About 0.1% to about 5% |
| Butylated Hydroxytoluene | Stabilizer | About 0 to about 1 % | About 0- about 1% | About 0- about 1% |
| Cetyl alcohol | Emollient | About 0 - about 10% | About 0 - about 10% | About 0 - about 10% |
| C12-15 alkyl benzoate | Emollient | About 0 - about 10% | About 0 - about 10% | About 0 - about 10% |
| Dimethicone | Spreading agent | About 0 to about 10% | About 0 to about 5% | About 2% |
| NaOH(10%) | Neutralizer | q.s. pH 5-8 | q.s. pH 5-8 | q.s. pH 5-8 |
| Steareth-10 | Polyoxyethylene alcohol Emulsifier | About 0 to about 5% | About 0 to about 5% | About 0 to about 5% |
| Sunscreen | Sunscreen | About 0 to about 10% | About 0 to about 10% | About 0 to about 10% |

The compositions of this invention may be administered topically, but may also be utilized in delivery of oral and parenteral formulations.

## Claims

1. A composition comprising an active agent selected from the group consisting of hydrophobic active agents and hydrophilic active agents and a combination thereof and a polymeric emulsifier and a sugar.

2. A topical skin composition according to claim 1, said composition further comprising a retinoid and a botanical derivative.

3. A composition according to claim 2 wherein the retinoid is retinol.

4. A composition according to claim 1 wherein the sugar is selected from the group consisting of ascorbic acid-2-glucoside, oligosaccharides and combinations thereof.

5. A composition according to claim 4 wherein said oligosaccharides are selected from the group consisting of lactose and melibiose, and combinations thereof.

6. A composition according to claim 4 wherein the sugar is ascorbic acid-2-glucoside.

7. A composition according to claim 2 wherein the polymeric emulsifier is a hydrophobically-modified acrylic acid.

8. A composition according to claim 7 wherein the hydrophobically-modified acrylic acid is acrylate/C10-30 alkyl acrylate cross-polymer.

9. A composition according to claim 2 wherein said botanical derivative is derived from a botanical family selected from the group consisting of leguminosae, solanaceae, gramineae, cucurbitacea, and combinations thereof.

10. A composition according to claim 9 wherein the botanical family is leguminosae.

11. A composition according to claim 10 wherein said botanical derivative of the leguminosae family group is selected from the group consisting of soybeans, limabeans, blackbeans, and combinations thereof.

12. A composition according to claim 9 wherein said botanical derivative comprises one or more isoflavones or derivatives thereof.

13. A composition according to claim 12 wherein said isoflavone derivatives are selected from the group consisting of daidzin, daidzein, genistin, genistein, and combinations thereof.

14. A composition according to claim 2 wherein said composition further comprises a polyoxyalkylene alcohol.

15. A composition according to claim 2 wherein said composition further comprises an ultraviolet screening agent.

16. A composition according to claim 15 wherein said ultraviolet screening agent is selected from the group consisting of a derivative of dibenzolymethane, octyl methoxycinnamate, and combinations thereof.

17. A topical skin composition, comprising: a retinoid, a sugar, a polymeric emulsifier, and a soy product.

18. A composition according to claim 17 wherein said retinoid is retinol.

19. A composition according to claim 17 wherein said sugar is selected from the group consisting of ascorbic acid-2-glucoside and oligosaccharides.

20. A composition according to claim 19 wherein said oligosaccharides are selected from the group consisting of lactose and melibiose, and combinations thereof.

21. A composition according to claim 19 wherein said sugar is ascorbic acid-2-glucoside.

22. A composition according to claim 17 wherein said polymeric emulsifier is a hydrophobically-modified acrylic acid.

23. A composition according to claim 22 wherein said hydrophobically-modified acrylic acid is acrylate/C10-30 alkyl acrylate cross-polymer.

24. A composition according to claim 17 wherein said soy product is a non-heat treated soy product.

25. A composition according to claim 24 wherein said non-heat treated soy product comprises a compound selected from the group consisting of soy milk, soy powder, soy protein, isoflavone, and combinations thereof.

26. A composition according to claim 17 further comprising a polyoxyalkylene alcohol.

27. A composition according to claim 17 further comprising a ultraviolet screening agent.

28. A composition according to claim 27 wherein the ultraviolet screening agent is selected from the group consisting of a derivative of dibenzolymethane, octyl methoxycinnamate, and combinations thereof.
